# EUROPEAN PATENT APPLICATION

(11) **EP 2 601 968 A1**
(43) Date of publication of application: **12.06.2013**
(21) Application number: 11009625.2
(22) Date of filing: 06.12.2011
(51) Int. Cl.: A61K 39/12, C07K 14/025, A61K 39/00, C12N 15/62

(54) **HPV derived polynucleic acids for therapy**

(71) Applicant: Deutsches Krebsforschungszentrum, 69120 Heidelberg (DE); King Saud University, 11451 Riyadh (SA)
(72) Inventor: Gissmann, Lutz., 69168 Wiesloch (DE); Öhlschläger, Peter, 52146 Würselen (DE); Senger, Tilo., 69121 Heidelberg (DE); Al-Majhdi, Fahad., Riyadh 11372 (SA); Kaufmann, Andreas., 12247 Berlin (DE); Kleinschmidt, Jürgen., 69245 Bammental (DE); Nieto, Karen, 69120 Heidelberg (DE)
(74) Representative: Schüssler, Andrea

(57) **Abstract**

Described are shuffled fragments of the genes encoding HPV 16 E6/E7 useful as a therapeutic vaccine against persistent HPV infections and HPV-related tumors, e.g, cervical carcinoma.

SEQUENCE LISTING

<110> Deutsches Krebsforschungszentrum

<120> HPV derived polynucleic acids for therapy

<130> K 3381EP

<160> 6

<170> BiSSAP 1.0

<210> 1
<211> 10
<212> PRT
<213> artificial sequences

<220>
<221> SOURCE
<222> 1..10
<223> /mol_type="protein"
      /note="HPV-16 E6 peptide 48-57 fragment"
      /organism="artificial sequences"

<400> 1
Glu Val Tyr Asp Phe Ala Phe Arg Asp Leu
1               5                   10

<210> 2
<211> 9
<212> PRT
<213> artificial sequences

<220>
<221> SOURCE
<222> 1..9
<223> /mol_type="protein"
      /note="HPV-16 E7 49-57 peptide "
      /organism="artificial sequences"

<400> 2
Arg Ala His Tyr Asn Ile Val Thr Phe
1               5

<210> 3
<211> 4791
<212> DNA
<213> artificial sequences

<220>
<221> source
<222> 1..4791
<223> /mol_type="DNA"
      /note="pTHkan vector"
      /organism="artificial sequences"

<400> 3
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttttgaga     240

tttctgtcgc cgactaaatt catgtcgcgc gatagtggtg tttatcgccg atagagatgg 300
cgatattgga aaaatcgata tttgaaaata tggcatattg aaaatgtcgc cgatgtgagt 360
ttctgtgtaa ctgatatcgc catttttcca aaagtgattt ttgggcatac gcgatatctg 420
gcgatagcgc ttatatcgtt tacgggggat ggcgatagac gactttggtg acttgggcga 480
ttctgtgtgt cgcaaatatc gcagtttcga tataggtgac agacgatatg aggctatatc 540
gccgatagag gcgacatcaa gctggcacat ggccaatgca tatcgatcta tacattgaat 600
caatattggc cattagccat attattcatt ggttatatag cataaatcaa tattggctat 660
tggccattgc atacgttgta tccatatcat aatatgtaca tttatattgg ctcatgtcca 720
acattaccgc catgttgaca ttgattattg actagttatt aatagtaatc aattacgggg 780
tcattagttc atagcccata tatggagttc cgcgttacat aacttacggt aaatggcccg 840
cctggctgac cgcccaacga cccccgccca ttgacgtcaa taatgacgta tgttcccata 900
gtaacgccaa tagggacttt ccattgacgt caatgggtgg agtatttacg gtaaactgcc 960
cacttggcag tacatcaagt gtatcatatg ccaagtacgc cccctattga cgtcaatgac 1020
ggtaaatggc ccgcctggca ttatgcccag tacatgacct tatgggactt tcctacttgg 1080
cagtacatct acgtattagt catcgctatt accatggtga tgcggttttg gcagtacatc 1140
aatgggcgtg gatagcggtt tgactcacgg ggatttccaa gtctccaccc cattgacgtc 1200
aatgggagtt tgttttggca ccaaaatcaa cgggactttc caaaatgtcg taacaactcc 1260
gccccattga cgcaaatggg cggtaggcgt gtacggtggg aggtctatat aagcagagct 1320
cgtttagtga accgtcagat cgcctggaga cgccatccac gctgttttga cctccataga 1380
agacaccggg accgatccag cctccgcggc cgggaacggt gcattggaac gcggattccc 1440
cgtgccaaga gtgacgtaag taccgcctat agagtctata ggcccacccc cttggcttct 1500
tatgcatgct atactgtttt tggcttgggg tctatacacc cccgcttcct catgttatag 1560
gtgatggtat agcttagcct ataggtgtgg gttattgacc attattgacc actcccctat 1620
tggtgacgat actttccatt actaatccat aacatggctc tttgccacaa ctctctttat 1680
tggctatatg ccaatacact gtccttcaga gactgacacg gactctgtat ttttacagga 1740
tggggtctca tttattattt acaaattcac atatacaaca ccaccgtccc cagtgcccgc 1800
agtttttatt aaacataacg tgggatctcc acgcgaatct cgggtacgtg ttccggacat 1860
gggctcttct ccggtagcgg cggagcttct acatccgagc cctgctccca tgcctccagc 1920
gactcatggt cgctcggcag ctccttgctc ctaacagtgg aggccagact taggcacagc 1980
acgatgccca ccaccaccag tgtgccgcac aaggccgtgg cggtagggta tgtgtctgaa 2040
aatgagctcg gggagcgggc ttgcaccgct gacgcatttg gaagacttaa ggcagcggca 2100
gaagaagatg caggcagctg agttgttgtg ttctgataag agtcagaggt aactcccgtt 2160
gcggtgctgt taacggtgga gggcagtgta gtctgagcag tactcgttgc tgccgcgcgc 2220

gccaccagac ataatagctg acagactaac agactgttcc tttccatggg tcttttctgc 2280

agtcaccgtc cttgacacga agctttctag agggccctat tctatagtgt cacctaaatg 2340

ctagagctcg ctgatcagcc tcgactgtgc cttctagttg ccagccatct gttgtttgcc 2400

cctcccccgt gccttccttg accctggaag gtgccactcc cactgtcctt tcctaataaa 2460

atgaggaaat tgcatcgcat tgtctgagta ggtgtcattc tattctgggg ggtggggtgg 2520

ggcaggacag caagggggag gattgggaag acaatagcag gcatgctggg gatgcggtgg 2580

gctctatggc ttctgaggcg gaaagaacca gctggggctc gaggggggat cgatcccgtc 2640

gacctcgaga gcttggcgta atcatggtca tagctgtttc ctgtgtgaaa ttgttatccg 2700

ctcacaattc cacacaacat acgagccgga agcataaagt gtaaagcctg gggtgcctaa 2760

tgagtgagct aactcacatt aattgcgttg cgctcactgc ccgctttcca gtcgggaaac 2820

ctgtcgtgcc agctgcatta atgaatcggc caacgcgcgg ggagaggcgg tttgcgtatt 2880

gggcgctctt ccgcttcctc gctcactgac tcgctgcgct cggtcgttcg gctgcggcga 2940

gcggtatcag ctcactcaaa ggcggtaata cggttatcca cagaatcagg ggataacgca 3000

ggaaagaaca tgtgagcaaa aggccagcaa aaggccagga accgtaaaaa ggccgcgttg 3060

ctggcgtttt tccataggct ccgcccccct gacgagcatc acaaaaatcg acgctcaagt 3120

cagaggtggc gaaacccgac aggactataa agataccagg cgtttccccc tggaagctcc 3180

ctcgtgcgct ctcctgttcc gaccctgccg cttaccggat acctgtccgc ctttctccct 3240

tcgggaagcg tggcgctttc tcaatgctca cgctgtaggt atctcagttc ggtgtaggtc 3300

gttcgctcca agctgggctg tgtgcacgaa ccccccgttc agcccgaccg ctgcgcctta 3360

tccggtaact atcgtcttga gtccaacccg gtaagacacg acttatcgcc actggcagca 3420

gccactggta acaggattag cagagcgagg tatgtaggcg gtgctacaga gttcttgaag 3480

tggtggccta actacggcta cactagaagg acagtatttg gtatctgcgc tctgctgaag 3540

ccagttacct tcggaaaaag agttggtagc tcttgatccg gcaaacaaac caccgctggt 3600

agcggtggtt tttttgtttg caagcagcag attacgcgca gaaaaaaagg atctcaagaa 3660

gatcctttga tcttttctac ggggtctgac gctcagtgga acgaaaactc acgttaaggg 3720

attttggtca tgagattatc aaaaaggatc ttcacctaga tccttttaaa ttaaaaatga 3780

agttttaaat caatctaaag tatatatgag taaacttggt ctgacaggaa ttcttagaaa 3840

aactcatcga gcatcaaatg aaactgcaat ttattcatat caggattatc aataccatat 3900

ttttgaaaaa gccgtttctg taatgaagga gaaaactcac cgaggcagtt ccataggatg 3960

gcaagatcct ggtatcggtc tgcgattccg actcgtccaa catcaataca acctattaat 4020

ttcccctcgt caaaaataag gttatcaagt gagaaatcac catgagtgac gactgaatcc 4080

ggtgagaatg gcaaaagttt atgcatttct ttccagactt gttcaacagg ccagccatta 4140

cgctcgtcat caaaatcact cgcatcaacc aaaccgttat tcattcgtga ttgcgcctga 4200

gcgagacgaa atacgcgatc gctgttaaaa ggacaattac aaacaggaat cgaatgcaac 4260

cggcgcagga acactgccag cgcatcaaca atattttcac ctgaatcagg atattcttct 4320

aatacctgga atgctgtttt cccggggatc gcagtggtga gtaaccatgc atcatcagga 4380

gtacggataa aatgcttgat ggtcggaaga ggcataaatt ccgtcagcca gtttagtctg 4440

accatctcat ctgtaacatc attggcaacg ctacctttgc catgtttcag aaacaactct 4500

ggcgcatcgg gcttcccata caatcgatag attgtcgcac ctgattgccc gacattatcg 4560

cgagcccatt tatacccata taaatcagca tccatgttgg aatttaatcg cggcctagag 4620

caagacgttt cccgttgaat atggctcatg gatccactct tcctttttca atattattga 4680

agcatttatc agggttattg tctcatgagc ggatacatat ttgaatgtat ttagaaaaat 4740

aaacaaatag gggttccgcg cacatttccc cgaaaagtgc cacctgacgt c 4791

<210> 4
<211> 720
<212> DNA
<213> Human papillomavirus

<220>
<221> source
<222> 1..720
<223> /mol_type="DNA"
      /note="HPV 16 E6"
      /organism="Human papillomavirus"

<400> 4
cccgccgcca ccatggataa agtgctgaac cgcgaagaaa gcctgcagct gatggatctg 60

ctgggcctgg aacgcagcgc gtggggcaac attccgctga tgcgcaaagc gtatctgaaa 120

aaatgcaaag aatttcatcc ggataaaggc ggcgatgaag aaaaaatgaa aaaaatgaac 180

accctgtata aaaaaatgga agatggcgtg aaatatgcgc atcagccgga ttttggcggc 240

tttatgcatc aaaagagaac tgcaatgttc caggaccccc aggagcggcc ccggaagctg 300

ccccagctgt gcaccgagct gcagaccacc atccacgaca tcatcctgga gtgcgtgtac 360

tgcaagcagc agctgctgcg gcgggaggtg tacgacttcg ccttccggga cctgtgcatc 420

gtgtaccggg acggcaaccc ctacgccgtg tgcgacaagt gcctgaagtt ctacagcaag 480

atcagcgagt accggcacta ctgctacagc ttgtacggca ccaccctgga gcagcagtac 540

aacaagcccc tgtgcgacct gctgatccgg tgcatcaact gccagaagcc cctgtgcccc 600

gaggagaagc agcggcacct ggacaagaag cagcggttcc acaacatccg gggccggtgg 660

accggccggt gcatgagctg ctgccggagc agccggaccc ggcgggagac ccagctgtaa 720

<210> 5
<211> 540
<212> DNA
<213> Human papillomavirus

<220>
<221> source
<222> 1..540
<223> /mol_type="DNA"
      /note="HPV 16-E7"
      /organism="Human papillomavirus"

<400> 5
cccgccgcca ccatggataa agtgctgaac cgcgaagaaa gcctgcagct gatggatctg      60

ctgggcctgg aacgcagcgc gtggggcaac attccgctga tgcgcaaagc gtatctgaaa     120

aaatgcaaag aatttcatcc ggataaaggc ggcgatgaag aaaaaatgaa aaaaatgaac     180

accctgtata aaaaaatgga agatggcgtg aaatatgcgc atcagccgga ttttggcggc     240

tttatgcacg gcgacacccc caccctgcac gagtacatgc tggacctgca gcccgagacc     300

accgacctgt actgctacga gcagctgaac gacagcagcg aggaggagga tgagatcgac     360

ggccccgccg gccaggctga gcccgaccgg gcccactaca acatcgtgac cttctgctgc     420

aagtgcgaca gcaccctgcg gctgtgcgtg cagagcaccc acgtggacat ccggaccctg     480

gaggacctgc tgatgggcac cctgggcatc gtgtgcccca tctgcagcca gaaaccctaa     540

<210> 6
<211> 1386
<212> DNA
<213> artificial sequences

<220>
<221> source
<222> 1..1386
<223> /mol_type="DNA"
      /note="HPV-16 E6E7 shuffeled"
      /organism="artificial sequences"

<400> 6
cccgccgcca ccatggataa agtgctgaac cgcgaagaaa gcctgcagct gatggatctg      60

ctgggcctgg aacgcagcgc gtggggcaac attccgctga tgcgcaaagc gtatctgaaa     120

aaatgcaaag aatttcatcc ggataaaggc ggcgatgaag aaaaaatgaa aaaaatgaac     180

accctgtata aaaaaatgga agatggcgtg aaatatgcgc atcagccgga ttttggcggc     240

tttatgcatc aaaagagaac tgcaatgttc caggaccccc aggagcggcc ccggaagctg     300

ccccagctgt gcaccgagct gcagaccacc atccacgaca tcatcctgga gtgcgtgaac     360

tgccagaagc ccctgtgccc cgaggagaag cagcggcacc tggacaagaa gcagcggttc     420

cacaacatcc ggggccggtg gaccggccgg tgcatgaagt gcctgaagtt ctacagcaag     480

atcagcgagt accggcacta ctgctacagc ttgtacggca ccaccctgga gcagcagtac     540

aacaagcccc tgtgcgacct gctgatccgg tgcatcatgc acggcgacac ccccaccctg     600

cacgagtaca tgctggacct gcagcccgag accaccgacc tgtactgcat ctgcagccag     660

aaacccaagt gcgacagcac cctgcggctg tgcgtgcaga gcacccacgt ggacatccgg     720

accctggagg acctgctgat gggcaccctg ggcatcgtgt gcccctactg caagcagcag 780

ctgctgcggc gggaggtgta cgacttcgcc ttccgggacc tgtgcatcgt gtaccgggac 840

ggcaacccct acgccgtgtg cgacagctgc tgccggagca gccggacccg gcgggagacc 900

cagctgattc atgatattat tcttgaatgt gtttattgta aacaacaact tcttcgtcgt 960

gaagatggta atccttatgc tgtttgtgat aaatgtctta aattttattc taaaattctt 1020

tgtgatcttc ttattcgttg tattaattgt caaaaacctc tttgtcctga acgtggtcgt 1080

tggactggtc gttgtatgtc ttgttgtcgt tcttctcgta ctcgttacga gcagctgaac 1140

gacagcagcg aggaggagga tgagatcgac ggccccgccg gccaggctga gcccgaccgg 1200

gcccactaca acatcgtgac cttctgctgc caaccagaga caactgatct ctactgttat 1260

gagcaattaa atgacagctc agagcattac aatattgtaa ccttttgttg caagtgtgac 1320

tctacgcttc ggttgtgcat gggcacacta ggaattgtgt gccccatctg ttctcagaaa 1380

ccataa 1386

## Description

The present invention relates to rearranged ("shuffled") fragments of the genes encoding HPV 16 E6/E7 useful as a therapeutic vaccine against HPV-related tumors as well as against persistent HPV infection.

Several million people fall ill with, and die of, carcinomas world-wide every year. These mortality rates have remained unchanged for many years despite intensive therapy research. Until now, patients suffering from carcinomas often have to undergo carcinoma-removing surgery and/or chemotherapy and/or radiation therapy. However, this is accompanied by very massive side-effects which then contribute to the mortality rates of patients suffering from carcinomas. Interestingly, human papillomaviruses (HPV) are associated with the development of over 5% of all cancers (Parkin and Bray, 2006). Prophylactic HPV vaccination is already available but shows no therapeutic effects in already infected people (Hildesheim et al., 2007). More precisely, contrary to the outstanding performance of the two commercially available prophylactic HPV vaccines designed to prevent HPV infection and HPV-related anogenital cancer (Cervix, vulva, penis, anus) and their precursors, therapeutic vaccines for the treatment of existing infections and disease derived therefrom are so far of limited success (for review see Albers and Kaufmann, 2009). The only promising therapeutic HPV vaccine consists of long overlapping peptides spanning the early proteins E6 and E7 of HPV 16 and was reported to yield a successful treatment of women with precursors to HPV 16-related vulval cancer (Kenter et al., 2009), however, at the expense of considerable side effects due to the use of an aggressive ajuvant.

Thus, there is a need of novel treatment options regarding HPV-associated tumors and persistent HPV infections which are effective and show fewer side effects.

According to the invention the solution of this technical problem is achieved by the subject matters defined in the claims.

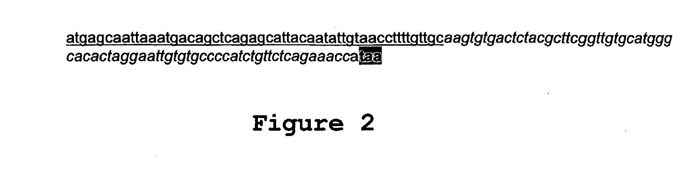

The experiments resulting in the present invention started from the accepted fact that inoculation of an oncogene (HPV 16 E6 and E7) into humans is ethically not feasible. Thus, the concept of shuffling of HPV 16 oncogenes was developed. This was based on the hypothesis that rearranging (shuffling) of segments of HPV oncogenes E6 and E7 will yield proteins that are devoid of detectable transforming activity due to loss of the authentic protein conformation. On the other hand the (CD8+ T cell-dependent) antiviral activity should remain unaltered since it requires linear epitopes presented via MHC class I complexes. In order to avoid loosing putative epitopes that might be located at the junctions of the original segments the sequences that span the original junctions were added as "appendix" at the end of the reshuffled segments ("core"). The constructs would require several recombinational events to regenerate a transformation competent wild-type gene. To further reduce the risk for recombination back to the wild type different codons were used for the core and appendix. In fact, none of the constructs that have been tested in standard transformation assays showed any activity above background; the immunologic properties were as expected. 8 individual HPV 16 E6/E7 shuffled genes were generated and their anti-HPV 16 E6/E7 activity was analyzed after immunization of C57/B6 mice by Elispot (gamma-interferon and granzyme, ⁵¹Cr-relase assay and rejection of HPV-16 positive tumor cells (two independent experiments in all instances). All constructs showed a satisfactory effect. In fact one construct ("E6E7 mixed shuffled"; see Figure 2) proved to be about 2fold more active in all assays as compared to the second most active construct as well as compared to all other shuffled constructs on average (statistically significant in all cases; see Tables 1 and 2).

### Brief description of the drawings

Figure 1: Nucleotide sequence of the vector pTHkan (4791 bp) The site of the insertion of the foreign (HPV) gene fragments is indicated (AAGCTT-HPV-SH-GEN-TCTAGA). In addition, the complete structure of pTH_kan_HPV_16SH is shown schematically.
Figure 2: Nucleotide sequence of HPV-16 E6, HPV 16-E7 and E6E7 mixed shuffled
Figure 3: *Ex vivo* IFN-γ Elispot responses after DNA immunization For details see Example 2.
Figure 4: *Ex vivo* GranzymeB Elispot responses after DNA immunization Four mice per group were immunized once i.m. with 100 µg empty vectors (pTHkan) or with HPV-16-encoding vectors as indicated. Given are two independent performed immunization experiments and the mean no. of GranzymeB secreting cells / 1 x 10⁴ splenocytes ± SD.
Figure 5: Cellular immune responses of AAV-E6/E7 sh ("mixed shuffled") in C57BL/6 See Example 7 for experimental details. Units on the Y-axis are IFN-γ secreting cells per 10⁴ splenocytes (left bars: E6; right bars: E7).

The present invention, thus, relates to an HPV derived polynucleic acid comprising
(a) a nucleic acid sequence as depicted in Figure 2;
(b) a nucleic acid sequence encoding a polypeptide being functionally equivalent to a polypeptide being encoded by a nucleic acid sequence of (a) ; or
(c) a fragment of a nucleic acid sequence of (a) or (b) and encoding a polypeptide being functionally equivalent to a polypeptide being encoded by a nucleic acid sequence of (a) or (b) .

Preferably, the nucleic acid sequence (b) shows an identity of at least 60%, preferably 70%, more preferably 80% to a nucleic acid sequence of (a). Particularly preferred is an identity of at least 90% or 95%. The term "functionally equivalent" as used herein relates to nucleic acid sequences of (b) or fragments of (a) or (b) encoding polypeptides which are still capable of inducing an immune response against an HPV E6 and/or E7 protein in a suitable host which is substantially identical to an immune response induced by a nucleic acid sequence as depicted in Figure 2. Preferably, said immune response is a T cell dependent immune response, more preferably a CD8+ dependent immune response.

Functional equivalence, i.e. induction of an immune response, is preferably determined using the methods described herein below (Examples 1 and 3 to 7). Functional equivalence can e.g. be tested using an ELISPOT assay as described in Example 1.

Alternatively, functional equivalence can be assayed by measuring the *ex vivo* IFN-γ Elispot response after immunization according to Example 3.

Alternatively, functional equivalence can be assayed by measuring the ex *vivo GranzymeB Elispot* responses after immunization according to Example 4.

Alternatively, functional equivalence can be assayed by measuring the CTL activity against wildtype HPV-16 E7 according to Example 5.

The skilled artisan knows other methods of determining the capability of a given polypeptide or a nucleic acid encoding such polypeptide to elicit an immune response. It is also known in the art that the methods mentioned supra need not be mutually exclusive and how such methods can be adapted e.g. for high-throughput screening.

Preferably, the immune response establishes therapeutic immunity to an HPV-containing neoplasm. Establishment of therapeutic immunity can e.g. be assayed by measuring the growth inhibition of C3 tumors in mice after immunization as described in Example 6; preferably, the nucleic acid sequences of (b) or fragments of (a) or (b) encode polypeptides which lead to an average tumor size after approximately seven weeks, preferably at day 39 or day 43, of less than 40 mm², less than 35 mm², less than 30 mm², less than 25 mm², or less than 20 mm².

The polypeptides encoded by the nucleic acid sequences of (b) are characterized by amino acid deletions, substitutions, and/or additions and show at least 70%, preferably 80%, and, more preferably, 90% identity to the proteins encodes by the nucleic acid sequence shown in Figure 2. Particularly preferred is an identity of 95 or 98%. Preferably, amino acid differences are due to one or more conservative amino acid substitutions. The term "conservative amino acid substitutions" involves replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

For the generation of polypeptides showing a particular degree of identity, e.g., genetic engineering can be used to introduce amino acid changes at specific positions of a cloned DNA sequence to identify regions critical for peptide function. For example, site directed mutagenesis or alanine-scanning mutagenesis (introduction of single alanine mutations at every residue in the molecule) can be used (Cunningham and Wells, 1989). The resulting mutant molecules can then be tested for immunogenicity using the assays of the examples.

In the fragment of the original polynucleic acid sequence at least 50%, preferably at least 60% contiguous aa, more preferably at least 70% contiguous aa and even more preferably at least 80% contiguous aa of the particular amino acid sequence are left. Such fragment is still useful as an efficient therapeutic vaccine.

The present invention also provides a vector containing an HPV derived polynucleic acid of the present invention. The direct injection of genetic material into a living host causes a small amount of its cells to produce the introduced gene products. This inappropriate gene expression within the host has important immunological consequences, resulting in the specific immune activation of the host against the gene delivered antigen. Direct injection of naked vector DNA induces strong immune responses to the antigen encoded by the gene vaccine. Once the plasmid DNA construct is injected the host cells take up the foreign DNA, expressing the viral gene and producing the antigen inside the cell. This form of antigen presentation and processing induces both MHC and class I and class II restricted cellular and humoral immune responses. The DNA vaccines are composed of vectors normally containing two units: the antigen expression unit composed of promoter/enhancer sequences, followed by antigen (FSP)-encoding and polyadenylation sequences and the production unit composed of sequences necessary for vector amplification and selection. The construction of vectors with vaccine inserts is accomplished using recombinant DNA technology and the person skilled in the art knows vectors that can be used for this approach. The efficiency of DNA immunization can be improved by stabilising DNA against degradation, and increasing the efficiency of delivery of DNA into antigen presenting cells. This has been demonstrated by coating biodegradable cationic microparticles (such as poly(lactide-co-glycolide) formulated with cetyltrimethylammonium bromide) with DNA. Such DNA-coated microparticles can be as effective at raising CTL as recombinant vaccinia viruses, especially when mixed with alum. Particles 300 nm in diameter appear to be most efficient for uptake by antigen presenting cells.

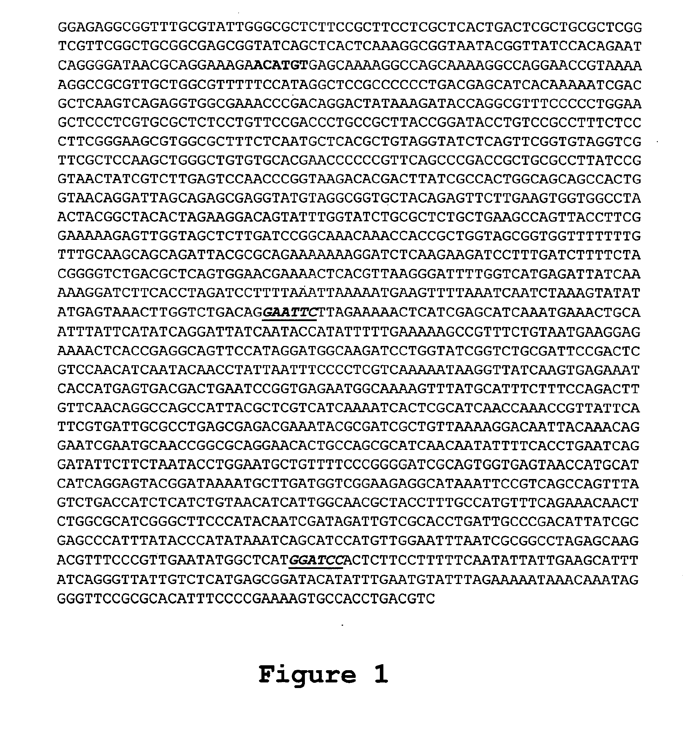

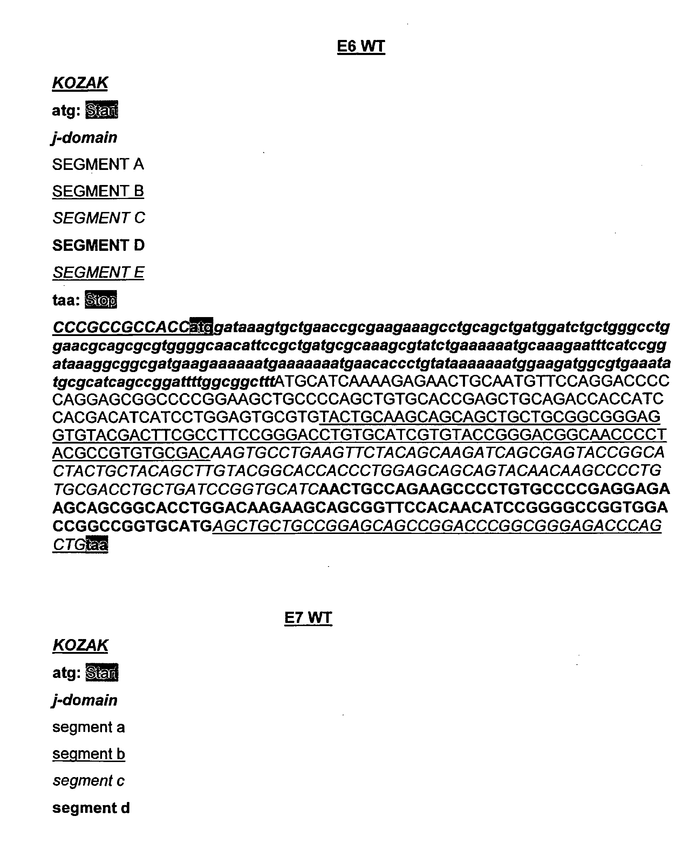

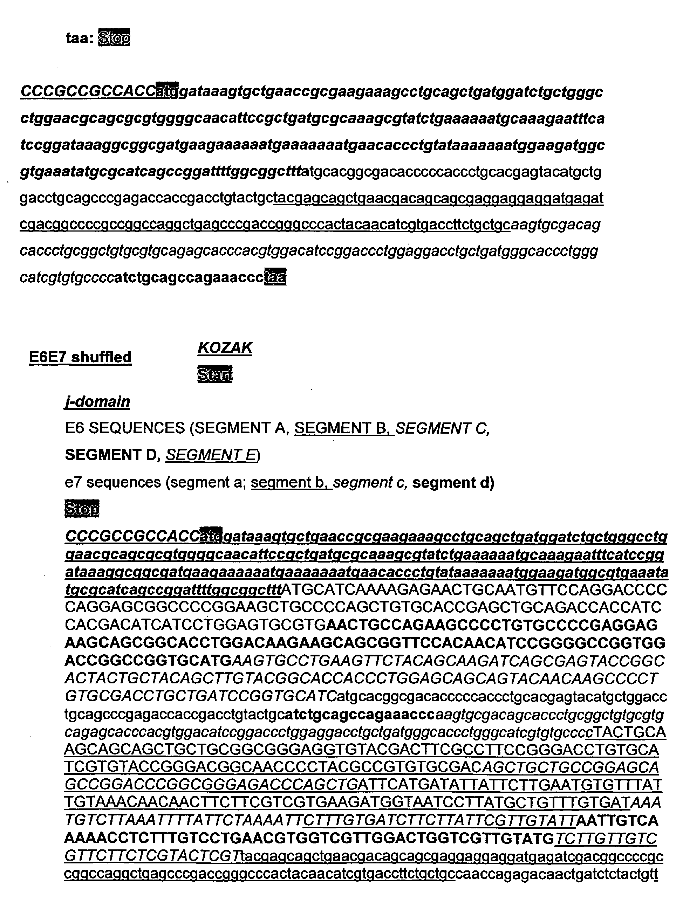

A variety of expression vectors, e.g., plasmids or viral vectors, may be utilised to contain and express polynucleic acid sequences of the present invention. A preferred plasmid is pTHkan having the nucleotide sequence shown in Figure 1.

A preferred viral vector is a poxvirus, adenovirus, retrovirus, herpesvirus or adeno-associated virus (AAV).

Particularly preferred AAV are AAV5 and AAV9.

Particularly preferred poxviruses are a vaccinia virus, NYVAC, avipox virus, canarypox virus, ALVAC, ALVAC(2), fowlpox virus or TROVAC.

Recombinant alphavirus-based vectors have also been used to improve DNA vaccination efficiency. A polynucleic acid of the invention is inserted into the alphavirus replicon, replacing structural genes but leaving non-structural replicase genes intact. The Sindbis virus and Semliki Forest virus have been used to build recombinant alphavirus replicons. Unlike conventional DNA vaccinations, however, alphavirus vectors are only transiently expressed. Alphavirus replicons raise an immune response due to the high levels of protein expressed by this vector, replicon-induced cytokine responses, or replicon-induced apoptosis leading to enhanced antigen uptake by dendritic cells.

The present invention also provides a vaccine containing a polynucleic acid, vector or polypeptide according to the present invention in an amount suitable for immunization of an individual and, preferably, one or more common auxiliary agents. Such a polynucleic acid, vector or polypeptide can be present as such or in combination with carriers. It is favourable for the carriers in the individual not to be immunogenic. Such carriers may be the individual's own proteins or foreign proteins or fragments thereof. Carriers, such as serum albumin, fibrinogen or transferrin or a fragment thereof are preferred. The fragments contain epitopes which are recognized by cytotoxic T cells, e.g. CD8⁺ T cells, and may induce a cytotoxic immune response. Such epitopes of cell cycle regulatory proteins can be determined by methods with which a person skilled in the art is familiar. It can also be advantageous that various fragments are simultaneously present. For the recombinant production of the above fragments, reference is made, e.g., to Sambrook et al., Molecular Cloning: A Laboratory Manual, 2nd edition, Cold Spring Harbor Laboratory Press, Cold Spring Harbor N.Y. (1989).

The present invention also relates to the use of the compounds of the present invention for the production of a vaccine for treating (a) a persistent HPV infection, preferably a symptomless persistent HPV infection, (b) an HPV-containing pre-neoplasia, neoplasia or carcinoma (including an advanced carcinoma) or (c) a pre-neoplasia, neoplasia or carcinoma (including an advanced carcinoma) expressing HPV E6 and/or E7.

For example, these may be anogenital carcinomas, in particular cervical carcinoma. Likewise benign modifications such as papillomas, adenomas, hyperplasias or similar proliferations of epithelial, mesenchymal or hematopoietic proliferations are also to be counted there among.

The employed term "individual" comprises an individual of any kind and being able to fall ill with carcinomas. Examples of such individuals are humans and animals as well as cells thereof.

The employed term "amount suitable for immunization of an individual" comprises any amount of a fragment of the invention, to which the above explanations apply correspondingly and with which an individual can be immunized. For example, the amount depends on whether immunization is intended as a prophylactic or therapeutic treatment. In addition, the individual's age, sex and weight play a role for determining the amount. It is favourable to give the individual 100 µg to 1 g of a fragment by means of injection. The injection may be made at various sites of the individual intramuscularly, subcutaneously, intradermally or in any other form of application (e.g. by the aid of electroporation). It may also be favourable to carry out one or more "booster injections" having about equal amount. In a preferred embodiment, different forms of application are used sequentially, more preferably priming by DNA and boosting by recombinant viral vector carrying the identical DNA sequence.

The employed term "common auxiliary agents" comprises any auxiliary agents suitable for a pharmaceutical composition to immunize an individual. Such auxiliary agents are, e.g., immunization adjuvants, such as GM-CSF or Freund's adjuvant, buffered common salt solutions, aluminium salts, (e.g. amorphous Aluminum Hydroxyphosphate, aluminum hydroxide + (MPL)detoxified bacterial lipids (e.g. 3-deacetylated monophosphoryl Lipid A, water, emulsions, such as oil/water emulsions, wetting agents, sterile solutions, etc.

By means of the present invention it is possible to immunize individuals, in particular humans and animals. Immunization takes place in order to induce cytotoxic T cells and also antibodies. Thus, it is possible to take therapeutic steps against persistent HPV infections, pre-neoplasias, neoplasias and carcinomas.

To further improve the efficacy of a vaccine which is based on the above described reshuffled gene segments of the invention with less severe side effects a heterologous prime-boost scheme can be applied, i.e. a priming by DNA immunization followed by a protein boost. In case of other vaccines this protocol appears to be superior as compared to a homologous immunization strategy (e.g. protein-protein; for review see Nieto et al., 2010).

The invention is explained by the below examples.

### Example 1

### Materials and Methods

### (A) Mice

C57BL/6 (H-2b) female mice at 6-8 weeks of age were purchased from Charles River Wiga (Sulzfeld, Germany) and kept in an isolator at the animal facilities of the German Cancer Research Center (Heidelberg, Germany). The mice were housed in accordance to the institutional guidelines.

### (B) Cell lines and culture conditions

293T cells were maintained in Dulbecco modified Eagle medium (DMEM) supplemented with 10% heat-inactivated fetal-calf serum, 100 U of penicillin/ml, and 100 µg of streptomycin/ml at 37°C in 5% CO₂.

### (C) Vaccine vector production and purification

All AAV vectors used in this study were produced as described previously (Nieto et al., 2009). Briefly, 293T cells were cotransfected with the packaging plasmid to produce pseudotyped AAV2/5, (pDP5) (Grimm et al., 2003) and AAV2/9 (Gao et al., 2002) and the vector plasmid UF2 Green Fluorescent Protein (GFP). Cells were incubated for 48 h at 37°C and 5% CO₂ collected, dispersed in lysis buffer (50 mM Tris-HCl, 150 mM NaCl, 2 mM MgCl₂ [pH 8.0]), and disrupted by three freeze-thaw cycles using liquid nitrogen. The tissue debris was removed by centrifugation (5,000 rpm, 20 min, 4°C), and the supernatant was digested with Benzonase (50 U/ml; Sigma, Taufkirchen, Germany) for 30 min at 37°C. AAV particles were purified from the cell lysates via an iodixanol step gradient followed by continuous gradient (Nieto et al., 2009). Virus stock was concentrated in a Vivaspin 20 (Vivascience, Gottingen, Germany) concentrator according to the manufactures protocol to a titer of 5 x10¹² genome-containing particles (gp)/ml and stored frozen at -80°C. Vector genome titers were determined by quantitative real-time PCR as described previously in Gao et al., 2004.

### (D) Immunization of mice

Female mice were kept under deep ketamine 10% / rompun 2% anesthesia by intraperitoneal injection.

Intranasal immunization: 10 ul of viral vector were delivered dropwise into one nostril. Seven days later a boost was administrated in the same way.

Intramuscular immunization: after shaving one leg skin, viral vector was once injected into the tibia anterior muscle of the right leg in a final volume of 50 µl.

Tattooed DNA immunization: DNA plasmid was delivered in 10 µl TE buffer one or two drops to the shaved skin at the dorsum followed by tattoo with a 7-linear tattoo needle using a commercial tattoo machine (Rotary 12000 PL, Bortech Tattoogrosshandel, Wuppertal, Germany). Six weeks after the first vaccination animals were sacrificed and spleens were isolated.

### (E) ELISPOT assay

The number of E6 and E7-specific CTLs was determined by IFN-γ ELISPOT assay. Sterile 96 well plates (MAHAS4510; Millipore, Eschborn, Germany) were equilibrated with PBS and coated with 200 ng of anti-mouse gamma interferon (IFN-γ) capture antibody (clone R4-6A2; BD Pharmingen, Heidelberg, Germany) in 100□l of PBS overnight at 4°C. Next day unbound antibody was removed and plates were washed once with PBS. After blocking for 2 h with RPMI medium containing 10% fetal calf serum and antibiotics at 37°C, splenocytes were seeded in quadruplicates in serial 2-fold dilutions ranging from 200,000 to 25,000 cells per well. For each quadruplicate, splenocytes in one well were left untreated (background control), in the next well cells were stimulated with 0.2 µg/well pokeweed mitogen (Sigma) as a positive control, next well cells were treated with 0.1 µM of HPV-16 E6 ₄₈EVYDFAFRDL₅₇ (Peng et al., 2004) peptide and in the last well treated with HPV-16 E7 ₄₉RAHYNIVTF₅₇ (Feltkamp et al., 1993) peptide. After 24 h incubation at 37°C cells were removed and plates were washed five times with PBS-0.01% Tween 20. Then, 20 ng of sterile-filtered biotinylated anti-mouse IFN-γ detection antibody (clone XMG1.2; BD Pharmingen) was added per well, and the plates were kept at 4°C overnight. Subsequently, plates were washed 4 times with PBS-0.01% Tween 20, and 100 µl/well streptavidin alkaline phosphatase (BD Pharmingen) diluted 1:1,000 in PBS for 30 min at room temperature and then washed three times with PBS-0.01% Tween 20, followed by three washing steps with PBS alone. Plates were developed with 75 µl/well BCIP/NBT (Sigma). After 1-5 min, the reaction was stopped by rinsing the plates with water. Evaluation was performed by counting the spots in an ELISPOT reader (ELISPOT Reader System ELR02; AID GmbH, Germany). Numbers of specific spots per 10⁶ cells were determined for each mouse by subtracting the number of spots of the internal negative control (cells incubated with medium alone) from the number of spots after incubation with the appropriate antigen-specific peptide.

### Example 2

### Construction of the vector used for expression of the HPV derived polynucleic acid sequences

The vector pTHamp (Hanke et al., (1998) was used as the starting vector. pTHamp was PCR amplified eliminating the gene for amp-resistance. The recognition sites for the restriction enzymes BamHI (GGATCC) and EcoRI (GAATTC) were inserted into the vector backbone. Simultaneously, the gene for kan-resistence was amplified from vector pET24a (Merck, Darmstadt, Germany) and the primers used for PCR also generated recognition sites for BamHI and EcoRI. Finally, the amplified sequence "kan-resistance gene" was inserted into the vector via the recognition sites for BamHI and EcoRI. The vector obtained was named "pTHkan".

The recognition sites for HindIII (AAGCTT) and XbaI (TCTAGA) were used for inserting the various new HPV-16 SH genes; see Figure 1. The 5'-3'-SV40 sequence (with SV40 enhancer sequences being modified in such a way that they cannot be translated) is inserted into pTHkan via the recognition site for PciI (ACATGT). The nucleotide sequence of HPV-16 E6E7 mixed shuffled construct of the present invention inserted into pTHkan is shown in Figure 2 and comprises the following sequences: "Kozak"-sequence, start-codon, J-domain, E6-shuffled, E7-shuffled, stop-codon.

### Example 3

### Ex vivo IFN-γ Elispot responses after DNA immunization

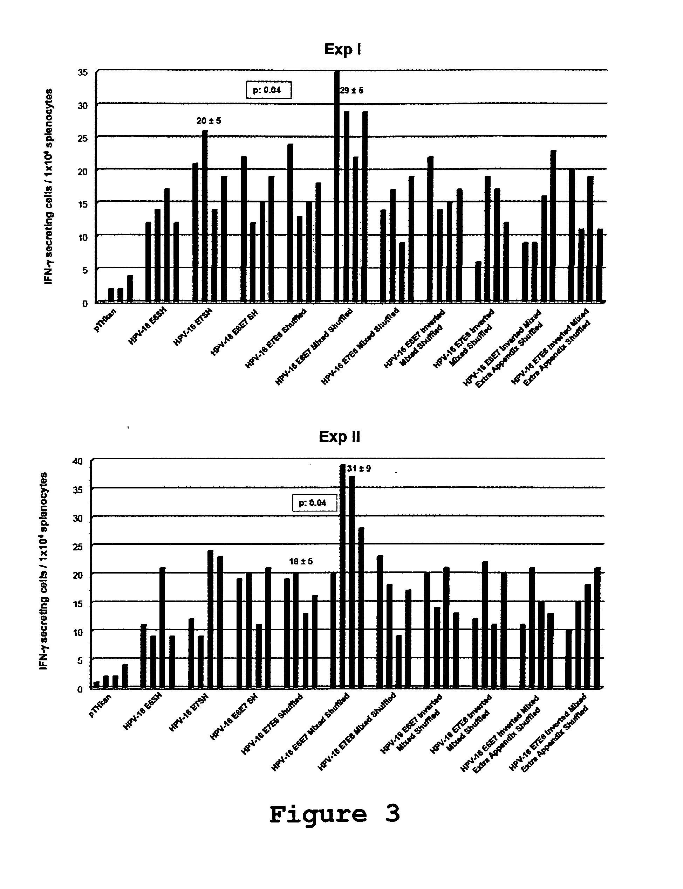

Four mice per group were immunized once i.m. with 100 µg empty vectors (pTHkan) or with HPV-16-encoding vectors as indicated. Given are two independent performed immunization experiments and the mean no. of IFN-γ secreting cells / 1 x 10⁴ splenocytes ± SD. The results are shown in Figure 3.

### Example 4

### Ex vivo GranzymeB Elispot responses after DNA immunization

Four mice per group were immunized once i.m. with 100 µg empty vectors (pTHkan) or with HPV-16-encoding vectors as indicated.

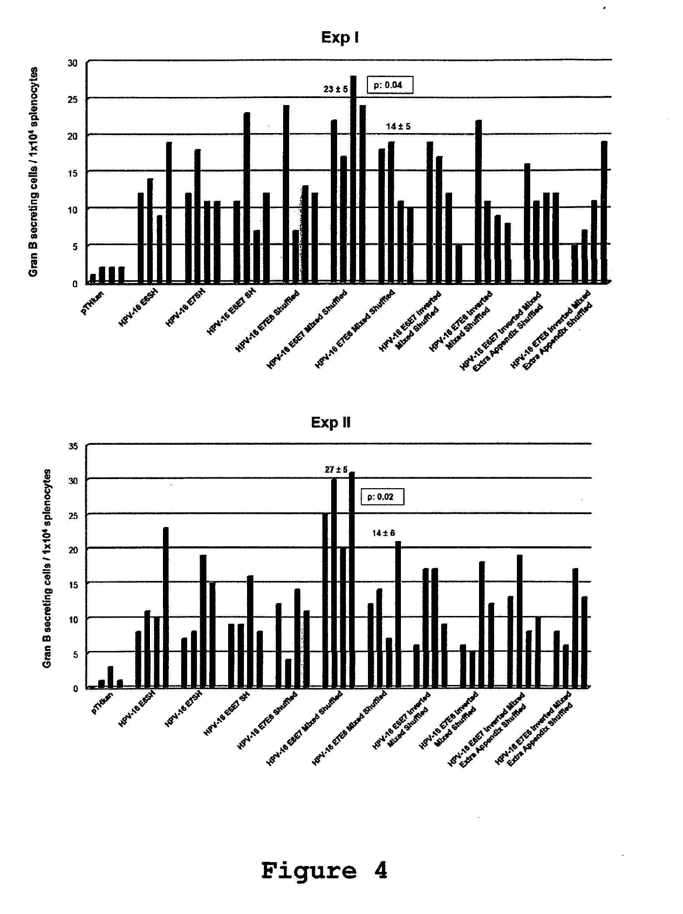

Given are two independent performed immunization experiments and the mean no. of GranzymeB secreting cells / 1 x 10⁴ splenocytes ± SD. The results are shown in Figure 4.

### Example 5

### CTL activity against wildtype HPV-16 E7

Animals (four per group) were treated once with empty vector (pTHkan) or with HPV-encoding plasmid as indicated and splenocytes were tested by ⁵¹Cr-release assays (Steinberg et al. (2005)) after one round of *in vitro* restimulation for lysis of syngeneic C3 cells. Given are two independently performed immunization experiments and the specific lysis ± SD. The results of Table 1 show that the construct of the invention is superior to the other constructs tested as regards lysis of syngeneic C3 cells (experiment 1: 69% +/- 19%, experiment 2: 78% +/- 19%).

**Table 1**

| ⁵¹**Cr-relase assay (after 1^{er} in vitro stimulation)** | **specific lysis (%) Exp I /Exp II** | |
|---|---|---|
| **pTHkan** | 4 ± 2 / 6 ± 2 | |
| **HPV-16 E6SH** | 12 ± 6 / 18 ± 5 | |
| **HPV-1**6 **E7SH** | 26 ± 9 / 28 ± 7 | |
| **HPV-16 E6E7 SH** | 33 ± 11 / 44 ± 14 | |
| **HPV-16 E7E6 SH** | 30 ± 14 / 43 ± 12 | |
| **HPV-16 E6E7 Mixed SH** | 69 ± 19 / 78 ± 19 | p: 0.04 |
| **HPV-16 E7E6 Mixed SH** | 39 ± 14 / 47 ± 16 | p: 0.04 |
| **HPV-16 E6E7 Inverted Mixed SH** | 17 ± 6 / 33 ± 17 | |
| **HPV-16 E7E6 Inverted Mixed SH** | 21 ± 9 / 36 ± 16 | |
| **HPV-16 E6E7 Inverted Mixed Extra Appendix SH** | 23 ± 11 / 29 ± 18 | |
| **HPV-16 E7E6 Inverted Mixed Extra Appendix SH** | 22 ± 12/32 ± 11 | |

### Example 6

### Growth of C3 tumors in mice after immunization with HPV-encoding vector

Mice (n=10/group) received tumor cells and were immunized twice with DNA (empty vector pTHkan, HPV-encoding vector plasmids showing various E6/E7-rearrangements as indicated) when the tumors were clearly palpable and surface tumor sizes were measured over time. Data gives the average tumor sizes ( S.D. at days 39 (first experiment) and 43 (second experiment) when the independent performed experiments terminated. The results shown in Table 2 provide evidence that the construct of the invention is superior to the other constructs tested as regards tumor regression after 39 and 43 days, respectively.

**Table 2**

| □ | | | | | | | | | | | |
|---|---|---|---|---|---|---|---|---|---|---|---|
| | **p: 0.03** | | | | | | | | | | |
| **Tumor Regression Exp I** | **pTHkan** | **E6SH** | **E7SH** | **E6E7 SH** | **E7E6 SH** | **E6E7 Mixed SH** | **E7E6 Mixed SH** | **E6E7 Inverted Mixed SH** | **E7E6 Inverted Mixed SH** | **E6E7 Inverted Mixed Extra Appendix SH** | **E7E6 inverted Mixed Extra Appendix SH** |
| **Average tumor size** at **day** 0 **(mm²) ± S.D.** | 4 ± 2 | 3 ± 2 | 3 ± 2 | 4 ± 2 | 3 ± 1 | 2 ± 1 | 4 ± 2 | 2 ± 1 | 3 ± 2 | 2 ± 1 | 3 ± 1 |
| **Average tumor size at day 39 (mm²) ± S.D** | 209 ± 77 | 84 ± 27 | 40 ± 17 | 33 ± 13 | 34 ± 12 | 20 ± 9 | 31 ± 12 | 39 ± 15 | 54 ± 17 | 44 ± 18 | 47 ± 20 |
| | | | | | | | | | | | |

| **Tumor Regression Exp II** | **pTHkan** | **E6SH** | **E7SH** | **E6E7 SH** | **E7E6 SH** | **E6E7 Mixed SH** | **E7E6 Mixed SH** | **E6E7 Inverted Mixed SH** | **E7E6 Inverted Mixed SH** | **E6E7 Inverted Mixed Extra Appendix SH** | **E7E6 inverted Mixed Extra Appendix SH** |
|---|---|---|---|---|---|---|---|---|---|---|---|
| **Average tumor size at day 0 (mm²) ± S.D.** | 2 ± 1 | 2 ± 1 | 4 ± 2 | 4 ± 2 | 2 ± 1 | 3 ± 1 | 3 ± 1 | 2 ± 1 | 2 ± 1 | 2 ± 1 | 4 ± 1 |
| **Average tumor size at day 43 (mm²) ± S.D**. | 297 ± 64 | 104 ± 44 | 59 ± 22 | 41 ± 19 | 40 ± 17 | 20 ± 14 | 39 ± 13 | 50 ± 21 | 70 ± 20 | 59 ± 17 | 64 ± 23 |
| | **p: 0.04** | | | | | | | | | | |

### Example 7

### Cellular immune responses of AAV-E6/E7 mixed shuffled (E6/E SH)in C57BL/6

A recombinant Adeno-associated virus (rAAVs) containing an HPV16 E6/E7 shuffled gene was generated. The respective shuffled gene was based on a construct tested for DNA vaccination which showed induction of E6- and E7-specific CTL responses. In this study vectors based upon AAV serotypes 5 and 9 were included to compare their ability for genetic, intranasal (i.n.) and intramuscular (i.m.) vaccination.

C57BL/6 mice (5 per group) were vaccinated i.n. with two doses of 5x10¹⁰ genome particles (gp) with rAAV5-E6/E7sh or rAAV9-E6/E7sh at a seven days interval. Intramuscular vaccination consists of one dose of 1x10¹¹ gp with rAAV9-E6/E7sh. Mice vaccinated i.n. or i.m. with rAAV9 expressing GFP (with 5x10¹⁰gp/dose) was used as negative controls. Three tattoo immunizations at a three days interval with HPV16 E6/E7sh (DNA) (50ug/dose) served as positive controls. Six weeks after the first immunization, the splenocytes were analyzed by an ex *vivo* IFNg-ELISPOT assay after stimulation with HPV16 E6 or E7 peptides.

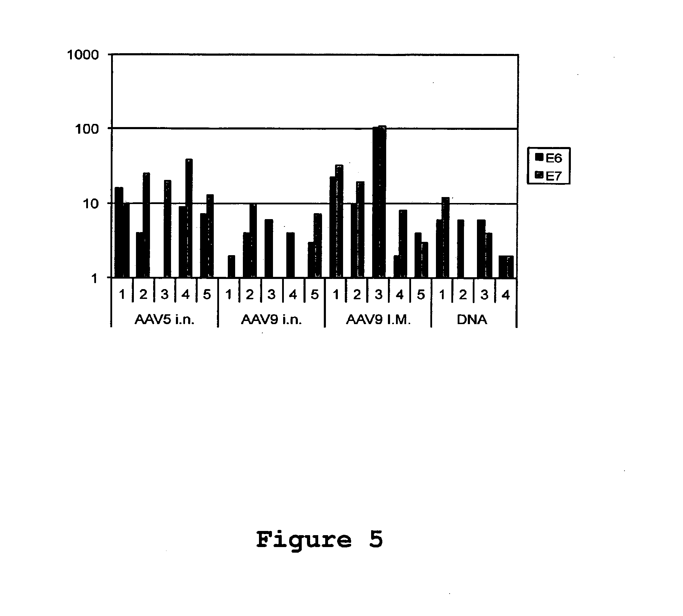

As shown in Figure 5 the cellular immune response seemed to be slightly weaker in animals vaccinated i.n. with rAAV9 vectors as compared to animals vaccinated i.n. with rAAV5 vectors. Nevertheless with a better response compared to the animals vaccinated with DNA HPV16 E6/E7sh. More interesting is the T-cell response, of animals vaccinated i.m. with rAAV9-E6/E7sh in that it led to highest titers of reactive T-cells and in that it led to vaccination against E6 and E7 with similar efficienies in almost all animals.

Taken together the results demonstrate that vaccination with rAAV-E6/E7sh induced more efficiently E6-specific and E7-specific T-cell responses than immunizations with E6/E7sh DNA. They also showed that delivery of rAAV9 intramuscular seemed to provide a stronger immune response than rAAV5.

### List of references

Albers and Kaufmann, Publ Health Genomics 2009,12:331.
Feltkamp et al., Eur J Immunol. 1993 Sep;23(9):2242-9.
Gao et al., Proc Natl Acad Sci U S A. 2002 ; 99(18):11854-9.
Gao et al., Journal of Virology 2004 Jun;78(12):6381-8.
Grimm et al., Molecular Therapy. 2003 Jun;7(6):839-50.
Hanke,T., et al., (1998). DNA multi-CTL epitope vaccines for HIV and Plasmodium falciparum: immunogenicity in mice. Vaccine 16(4), 426-35.
Hildesheim,A., Herrero,R., Wacholder,S., Rodriguez,A.C., Solomon,D., Bratti,M.C., Schiller,J.T., Gonzalez,P., Dubin,G., Porras,C., Jimenez,S.E., and Lowy,D.R. (2007). Effect of human papillomavirus 16/18 L1 viruslike particle vaccine among young women with preexisting infection: a randomized trial. JAMA 298, 743-753.
Kenter et al., NEJM 2009, 361:1838.
Nieto et al., Antiviral Therapy. 2009;14(8):1125-37.
Nieto et al., Antivir Ther. 2010, 15:951.
Parkin,D.M. and Bray,F. (2006). Chapter 2: The burden of HPV-related cancers. Vaccine 24 Suppl 3, S11-S25.
Peng et al., Journal Virology. 2004 Aug;78(16):8468-76.
Steinberg T, Ohlschlager P, Sehr P, Osen W, Gissmann L., Modification of HPV 16 E7 genes: correlation between the level of protein expression and CTL response after immunization of C57BL/6 mice. Vaccine 2005;23(9):1149-57.

GACGGATCGGGAGATCTCCCGATCCCCTATGGTCGACTCTCAGTACAATCTGCTCTGATGCCG
CATAGTTAAGCCAGTATCTGCTCCCTGCTTGTGTGTTGGAGGTCGCTGAGTAGTGCGCGAGCA
AAATTTAAGCTACAACAAGGCAAGGCTTGACCGACAATTGCATGAAGAATCTGCTTAGGGTTA
GGCGTTTTGCGCTGCTTCGCGATGTACGGGCCAGATATACGCGTTTTGAGATTTCTGTCGCCG
ACTAAATTCATGTCGCGCGATAGTGGTGTTTATCGCCGATAGAGATGGCGATATTGGAAAAAT
CGATATTTGAAAATATGGCATATTGAAAATGTCGCCGATGTGAGTTTCTGTGTAACTGATATC
GCCATTTTTCCAAAAGTGATTTTTGGGCATACGCGATATCTGGCGATAGCGCTTATATCGTTT
ACGGGGGATGGCGATAGACGACTTTGGTGACTTGGGCGATTCTGTGTGTCGCAAATATCGCAG
TTTCGATATAGGTGACAGACGATATGAGGCTATATCGCCGATAGAGGCGACATCAAGCTGGCA
CATGGCCAATGCATATCGATCTATACATTGAATCAATATTGGCCATTAGCCATATTATTCATT
GGTTATATAGCATAAATCAATATTGGCTATTGGCCATTGCATACGTTGTATCCATATCATAAT
ATGTACATTTATATTGGCTCATGTCCAACATTACCGCCATGTTGACATTGATTATTGACTAGT
TATTAATAGTAATCAATTACGGGGTCATTAGTTCATAGCCCATATATGGAGTTCCGCGTTACA
TAACTTACGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTGGAGTAT
TTACGGTAAACTGCCCACTTGGCAGTACATCAAGTGTATCATATGCCAAGTACGCCCCCTATT
GACGTCAATGACGGTAAATGGCCCGCCTGGCATTATGCCCAGTACATGACCTTATGGGACTTT
CCTACTTGGCAGTACATCTACGTATTAGTCATCGCTATTACCATGGTGATGCGGTTTTGGCAG
TACATCAATGGGCGTGGATAGCGGTTTGACTCACGGGGATTTCCAAGTCTCCACCCCATTGAC
GTCAATGGGAGTTTGTTTTGGCACCAAAATCAACGGGACTTTCCAAAATGTCGTAACAACTCC
GCCCCATTGACGCAAATGGGCGGTAGGCGTGTACGGTGGGAGGTCTATATAAGCAGAGCTCGT
TTAGTGAACCGTCAGATCGCCTGGAGACGCCATCCACGCTGTTTTGACCTCCATAGAAGACAC
CGGGACCGATCCAGCCTCCGCGGCCGGGAACGGTGCATTGGAACGCGGATTCCCCGTGCCAAG
AGTGACGTAAGTACCGCCTATAGAGTCTATAGGCCCACCCCCTTGGCTTCTTATGCATGCTAT
ACTGTTTTTGGCTTGGGGTCTATACACCCCCGCTTCCTCATGTTATAGGTGATGGTATAGCTT
AGCCTATAGGTGTGGGTTATTGACCATTATTGACCACTCCCCTATTGGTGACGATACTTTCCA
TTACTAATCCATAACATGGCTCTTTGCCACAACTCTCTTTATTGGCTATATGCCAATACACTG
TCCTTCAGAGACTGACACGGACTCTGTATTTTTACAGGATGGGGTCTCATTTATTATTTACAA
ATTCACATATACAACACCACCGTCCCCAGTGCCCGCAGTTTTTATTAAACATAACGTGGGATC
TCCACGCGAATCTCGGGTACGTGTTCCGGACATGGGCTCTTCTCCGGTAGCGGCGGAGCTTCT
ACATCCGAGCCCTGCTCCCATGCCTCCAGCGACTCATGGTCGCTCGGCAGCTCCTTGCTCCTA
ACAGTGGAGGCCAGACTTAGGCACAGCACGATGCCCACCACCACCAGTGTGCCGCACAAGGCC
GTGGCGGTAGGGTATGTGTCTGAAAATGAGCTCGGGGAGCGGGCTTGCACCGCTGACGCATTT
GGAAGACTTAAGGCAGCGGCAGAAGAAGATGCAGGCAGCTGAGTTGTTGTGTTCTGATAAGAG
TCAGAGGTAACTCCCGTTGCGGTGCTGTTAACGGTGGAGGGCAGTGTAGTCTGAGCAGTACTC
GTTGCTGCCGCGCGCGCCACCAGACATAATAGCTGACAGACTAACAGACTGTTCCTTTCCATG
GGTCTTTTCTGCAGTCACCGTCCTTGACACGAAGCTT-HPV-SH-GEN-
TCTAGAGGGCCCTATTCTATAGTGTCACCTAAATGCTAGAGCTCGCTGATC

AGCCTCGACTGTGCCTTCTAGTTGCCAGCCATCTGTTGTTTGCCCCTCCCCCGTGCCTTCCTT
GACCCTGGAAGGTGCCACTCCCACTGTCCTTTCCTAATAAAATGAGGAAATTGCATCGCATTG
TCTGAGTAGGTGTCATTCTATTCTGGGGGGTGGGGTGGGGCAGGACAGCAAGGGGGAGGATTG
GGAAGACAATAGCAGGCATGCTGGGGATGCGGTGGGCTCTATGGCTTCTGAGGCGGAAAGAAC
CAGCTGGGGCTCGAGGGGGGATCGATCCCGTCGACCTCGAGAGCTTGGCGTAATCATGGTCAT
AGCTGTTTCCTGTGTGAAATTGTTATCCGCTCACAATTCCACACAACATACGAGCCGGAAGCA
TAAAGTGTAAAGCCTGGGGTGCCTAATGAGTGAGCTAACTCACATTAATTGCGTTGCGCTCAC
TGCCCGCTTTCCAGTCGGGAAACCTGTCGTGCCAGCTGCATTAATGAATCGGCCAACGCGCGG

## Claims

1. An HPV derived polynucleic acid comprising
(a) a nucleic acid sequence as depicted in Figure 2;
(b) a nucleic acid sequence showing at least 60% identity to a nucleic acid sequence of (a) and encoding a polypeptide being functionally equivalent to a polypeptide being encoded by a nucleic acid sequence of (a); or
(c) a fragment of a nucleic acid sequence of (a) or (b) and encoding a polypeptide being functionally equivalent to a polypeptide being encoded by a nucleic acid sequence of (a) or (b).

2. A vector containing a polynucleic acid of claim 1.

3. The vector of claim 2, wherein said vector is a plasmid or viral vector.

4. The vector of claim 3, which is pTHkan.

5. The vector of claim 3, wherein the viral vector is Adeno-associated virus (AAV).

6. The AAV of claim 5 which is AAV5 or AAV9.

7. A host cell harbouring the vector of any one of claims 2 to 4.

8. A polypeptide being encoded by a polynucleic acid of claim 1.

9. A vaccine containing a polynucleic acid of claim 1, a vector of any one of claims 2 to 6, or a polypeptide according to claim 8 together with a pharmaceutically acceptable carrier.

10. A polynucleic acid of claim 1, a vector of any one of claims 2 to 6, or a polypeptide according to claim 8 for use in treating a persistent HPV infection, an HPV-containing pre-neoplasia, neoplasia or carcinoma.

11. A polynucleic acid of claim 1, a vector of any one of claims 2 to 6, or a polypeptide according to claim 8 for the use according to claim 10 **characterized in that** the use is for treating an advanced HPV-induced carcinoma.

12. A polynucleic acid of claim 1, a vector of any one of claims 2 to 6, or a polypeptide according to claim 8 for the use according to claim 10 or 11 **characterized in that** the use is for treating an anogenital carcinoma.

13. A polynucleic acid of claim 1, a vector of any one of claims 2 to 6, or a polypeptide according to claim 8 for the use according to claim 12 **characterized in that** the use is for treating a cervical carcinoma.

14. The polynucleic acid, vector, or polypeptide of claim 10 or 11 (bzw any one of claims 10 to 13), wherein the HPV is HPV 16, 18, 31, 33, 35, 52, 58, or 66.
